# EUROPEAN PATENT SPECIFICATION

(11) **EP 1 110 953 B1**
(45) Date of publication and mention of the grant of the patent: **28.10.2009**
(21) Application number: 01104696.8
(22) Date of filing: 06.06.1995
(51) Int. Cl.: C07D 239/94, C07D 491/04, A61K 31/505

(54) **Quinazoline derivatives**
Chinazolinone Derivate
Dérivés de quinazolinone

(30) Priority: 30.03.1995 US 413300
(43) Date of publication of application: 27.06.2001
(62) Divisional of application: 95918713.9
(73) Proprietor: Pfizer Products Inc., Groton, CT 06340 (US); OSI Pharmaceuticals, Inc., Melville, NY 11747 (US)
(72) Inventor: Schnur, Rodney Caughren, Noank, Connecticut 06340 (US); Arnold, Lee Daniel, Westborough, Massachusetts 01581 (US)
(74) Representative: Blakey, Alison Jane

(56) References cited:
- EP-A- 0 635 498

## Description

### Background of the Invention

This invention relates to intermediate compounds that may be used to produce 4-(substitutedphenylamino)quinazoline derivates which are useful in the treatment of hyperproliferative diseases, such as cancer, in mammals.

Many of the current treatment regimes for cancer utilize compounds which inhibit DNA synthesis. Such compounds are toxic to cells generally but their toxic effect on the rapidly dividing tumor cells can be beneficial. Alternative approaches to anti-cancer agents which act by mechanisms other than the inhibition of DNA synthesis have been explored in order to enhance the selectivity of action against cancer cells.

It is known that a cell may become cancerous by virtue of the transformation of a portion of its DNA into an oncogene (i.e. a gens which, on activation, leads to the formation of malignant tumor cells). Many oncogenes encode proteins which are aberrant tyrosine kinases capable of causing cell transformation. Alternatively, the overexpression of a normal proto-oncogenic tyrosine kinase may also result in proliferative disorders, sometimes resulting in a malignant phenotype.

Receptor tyrosine kinases are large enzymes which span the cell membrane and possess an extracellular binding domain for growth factors such as epidermal growth factor, a transmembrane domain, and an intracellular portion which functions as a kinase to phosphorylate specific tyrosine residues in proteins and hence to influence cell proliferation. It is known that such kinases are frequently aberrantly expressed in common human cancers such as breast cancer, gastrointestinal cancer such as colon, rectal or stomach cancer, leukemia, and ovarian, bronchial or pancreatic, cancer. It hasp also been shown that epidermal growth factor receptor (EGFR) which possessed tyrosine kinase activity is mutated and/or overexpressed in many human cancers such as brain, lung, squamous cell, bladder, gastric; breast, head and neck, oesophageal gynecological and thyroid tumors.

Accordingly, it has been recognized that inhibitors of receptor tyrosine kinases are useful as a selective inhibitors of the growth of mammalian cancer cells. For example, erbstatin, a tyrosine kinase inhibitor selectively attenuates the growth in athymic nude mice of a transplanted human mammary carcinoma which expresses epidermal growth factor receptor tyrosine kinase (EGFR) but is without effect on the growth of another carcinoma which does not express the EGF receptor.

Various other compounds, such as styrene derivatives, have also been shown to possess tyrosine kinase inhibitory properties. More recently five European patent publications, namely EP 0 566 226 A1, EP 0 602 851 A1, EP 0 635 507 A1, EP 0 635 498 A1 and EP 0 520 722 A1 have disclosed that certain quinazoline derivatives possess anti-cancer properties which result from their tyrosine kinase inhibitory properties. Also PCT publication WO 92/20642 discloses bis-mono and bicyclic aryl and heteroaryl.compounds as tyrosine kinase inhibitors.

Although the anti-cancer compounds described above make a significant contribution to the art there is a continuing search in this field of art for improved anti-cancer pharmaceuticals.

EP-A-0 790 986 which corresponds to WO 96/15118 is comprised within the state of the art in accordance with Article 54(3) EPC. Based on the disclosure provided in the earliest priority application GB 94228866.5, EP-A-0 790 986 discloses a large genus of intermediate compounds (represented by Formula II therein) for the preparation of aniline derivatives (represented by Formula I therein).

### Summary of the Invention

This invention is directed to compounds of the formula: 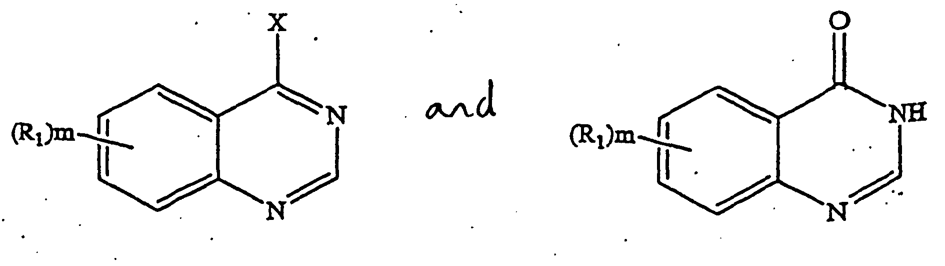 wherein
X is OH;
m is 2 or 3;
each R₁ is R₅O, where R₅ is (C₁-C₄) alkyl; wherein the alkyl moiety in R₅O is substituted with R₆O, where R₆ is hydrogen or R₅ and the compound 4-chloro-6,7-bis-(2-methoxy-ethoxy)-quinazoline.

The present invention also provides a process for preparing the compound 4-Chloro-6,7-bis-(2-methoxy-ethoxy)-quinazoline, which comprises treating the compound having the structure: 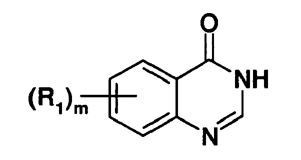 wherein m is 2 and each R₁ methoxy-ethoxy, with oxalylchloride in the presence of CHC1₃ and DMF.

Described herein is a process for preparing a compound of the formula 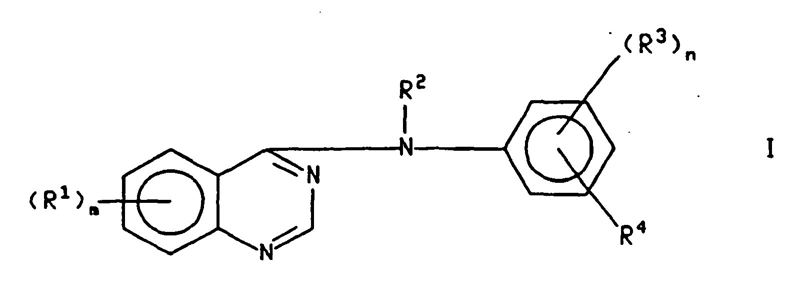 wherein
m is 1, 2, or 3;
each R¹ is independently selected from hydrogen, halo, hydroxy, amino, hydroxyamino, carboxy, (C₁-C₄)alkoxycarbonyl, nitro, guanidino, ureido, carbamoyl, cyano, trifluoromethyl, (AC⁶)₂N-carbonyl, and phenyl-W-alkyl wherein W is selected from a single bond, O, S and NH;
or each R¹ is independently selected from cyano-(C₁-C₄)-alkyl and R⁹ wherein R⁹ is selected from the group consisting of R⁵, R⁵O, (R⁶)₂N, R⁷C(=O), R⁵ONH, A and R⁵Y; wherein R⁵ is (C₁-C₄)alkyl; R⁶ is hydrogen or R⁵ wherein the R⁵s are the same or different; R⁷ is R⁵, R⁵O or (R⁶)₂N; A is selected from piperidino-, morpholino, pyrrolidino and 4-R⁶-piperazin-1-yl, imidazol-1-yl, 4-pyridon-1-yl, carboxy-(C₁-C₄)-alkyl, phenoxy, phenyl, phenylsulfanyl, (C₂-C₄)-alkenyl, (R⁶)₂-N-carbonyl-(C₁-C₄)-alkyl; and Y is selected from S, SO, SO₂; the alkyl moieties in (R⁶)₂N are optionally substituted with halo or R⁹ wherein R⁹ is defined as above, and the alkyl moieties in R⁵ and R⁵O are optionally substitute with halo, R⁶O or R⁹ wherein R⁹ and R⁶ are defined as above, and wherein the resulting groups are optionally substituted with halo or R⁹ with the proviso that a nitrogen, oxygen or sulfur atom and another heteroatom can not be attached to the same carbon atom, and with the further proviso that no more than three "A⁹" units may comprise R¹;
or each R¹ is independently selected from R⁶-sulfonylamino, phthalimido-(C₁-C₄)-alkylsulfonylamino, benzamido, benzenesulfonylamino, 3-phenylureido, 2-oxopyrrolidin-1-yl, 2.5-dioxopyrrolidin-1-yl, and R¹⁰-(C₂-C₄)-alkanoylamino wherein R¹⁰ is selected from halo, R⁶O, (C₂-C₄)-alkanoyloxy, R⁷C(=O), and (R⁶)₂N; and wherein said benzamido or benzenesulfonylamino or phenyl or phenoxy or anilino or phenylsulfanyl substituent in or any two R's taken together with the carbons to which they are attached comprise a 5-8 membered ring comprising at least one or two heteratoms selected from oxygen, sulfur or nitrogen; and wherein the alkyl groups and alkyl portions of the alkoxy or akylamino groups may be straight chained or if comprised of at least three carbons may be branched or cyclic;
R² is selected from hydrogen and optionally substituted (C₁-C₅)-alkyl;
n is 1 or 2 and each R² is independently selected from hydrogen, optionally substituted (C₁-C₆)-alkyl, optionally substituted amino, halo, hydroxy, optionally substituted hydroxy;
R⁴ is azido or R"-ethynyl wherein R¹¹ is selected from hydrogen, optionally substituted (C₁-C₆)alkyl wherein the substituents are selected from hydrogen, amino, hydroxy, R⁵O, R⁵NH and (R⁵)₂N.
which comprises
a) treating a compound of the formula 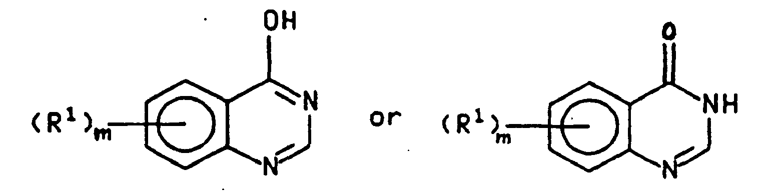 wherein R¹ and m are as defined above,
   with CCl₄ and an optionally substituted triarylphosphine, optionally supported on an inert polymer, of the formula Ar₃P wherein each Ar is an optionally substituted (C₅-C₁₀)aryl group and each of the substitutents is independently selected from (C₁-C₆)alkyl; and
b) treating the product of step a) with a compound of the formula 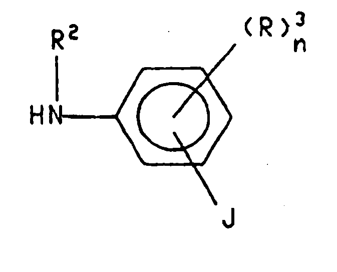 wherein R², R³ and n are as defined above, and J is Y or R⁴, wherein R⁴ is as defined above, with the proviso that when J is Y then the product of step b) must further be treated with an alkyne.

Described herein is also a method for treating a hyperproliferative disease in a mammal by administering to a mammal suffering from a hyperproliferative disease, a hyperproliferative disease treating amount of a Formula I

Also described herein are pharmaceutical compositions for the treatment of a hyperproliferative disease in mammals which comprise a hyperproliferative disease treating amount of a compound of the Formula I and a pharmaceutically acceptable carrier.

By halo is meant chloro, bromo, iodo, or fluoro.

By alkyl is meant straight chain, cyclic or branched saturated or unsaturated hydrocarbyl moiety with the proviso that said alkyl must comprise three or more carbon atoms if it is branched or cyclic.

As used herein, the expression "reaction-inert solvent" refers to a solvent which does not interact with starting materials, reagents, Intermediates or products in a manner which adversely affects the yield of the desired product.

Other features and advantages will be apparent from the specification and claims which describe the invention.

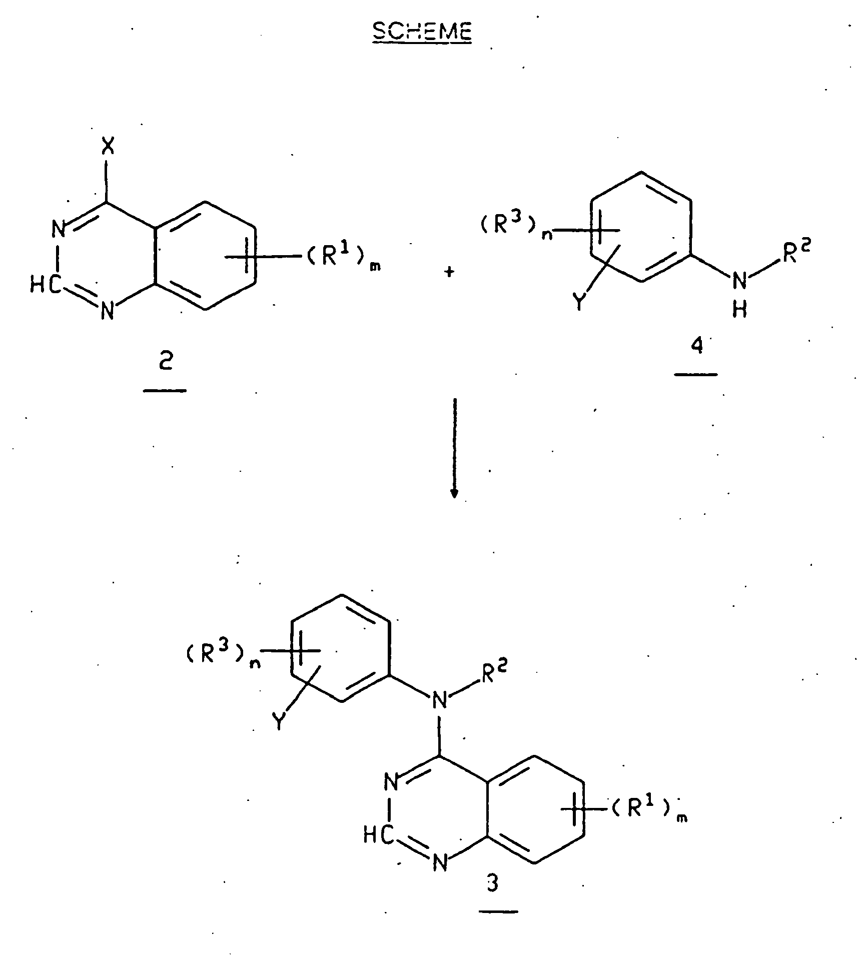

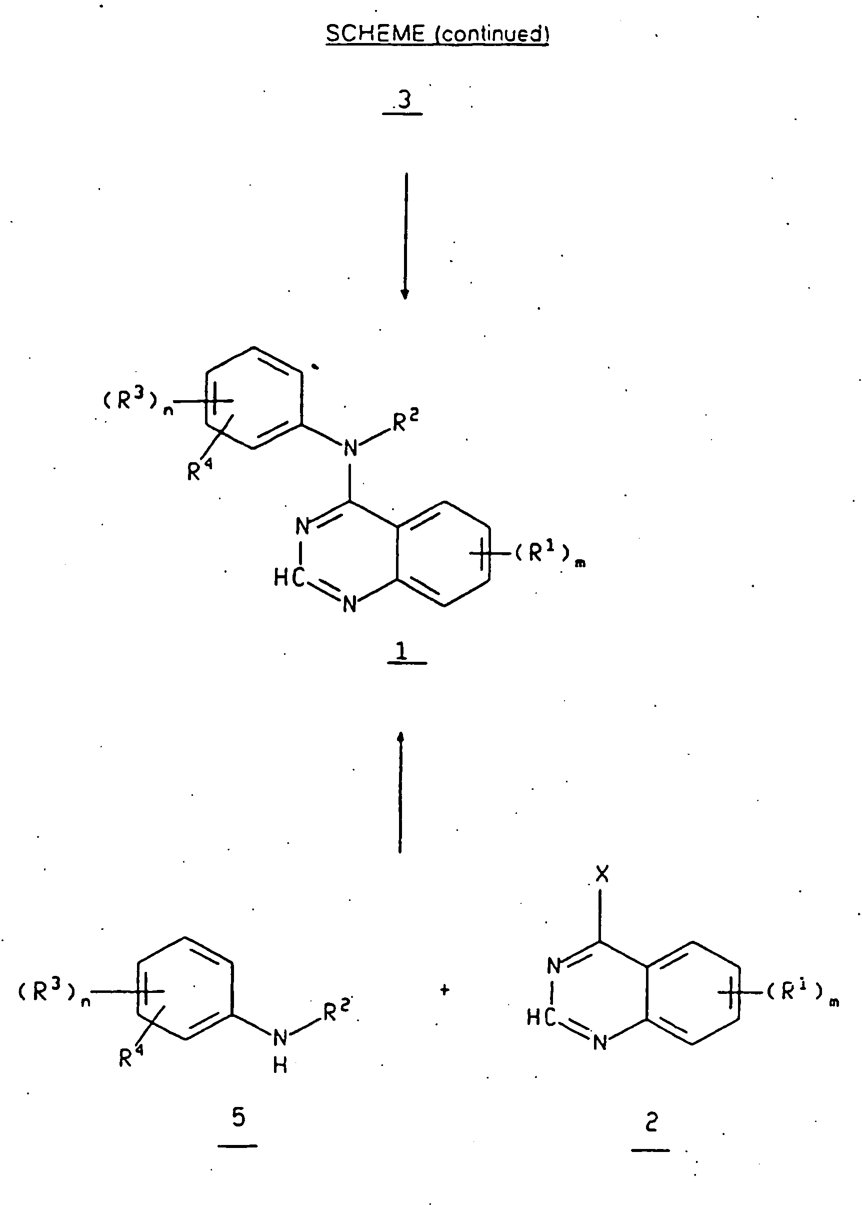

### Detailed Description of the Invention

The Formula I compounds, pharmaceutically acceptable salts and prodrugs thereof (hereafter the active compounds) may be prepared by any process known to be applicable to the preparation of chemically-related compounds.

In general the active compounds may be made from the appropriately substituted quinazoline using the appropriately substituted amine.

As shown in the.Scheme the appropriate 4-substituted quinazoline 2 wherein X is a suitable displaceable leaving group such as halo, aryloxy, alkylsulfinyl, alkylsulfonyl such as trifluoromethanesulfonyloxy, arylsulfinyl, arylsulfonyl, siloxy, cyano, pyrazolo, triazolo or tetrazolo, preferably a 4-chloroquinazoline, is reacted with the appropriate amine or amine hydrochloride 4 or 5, wherein R⁴ is as described above and Y is Br, I, or trifluoromethane-sulfonyloxy in a solvent such as a (C₁-C₆)alcohol, dimethylformamide (DMF), N-methylpyrrolidin-2-one, chloroform, acetonitrile, tetrahydrofuran (THF), 1-4 dioxane, pyridine or other aprotic solvent. The reaction may be effected in the presence of a base, preferably an alkali or alkaline earth metal carbonate or hydroxide or a tertiary amine base, such as pyridine, 2,6-lutidine, collidine, N-methyl-morpholine, triethylamine, 4-dimethylamino-pyridine or N,N-dimethylaniline. These bases are hereinafter refered to as suitable bases. The reaction mixture is maintained at a temperature from about ambient to about the reflux temperature of the solvent, preferably from about 35°C to about reflux, until substantival no remaining 4-haloquinazoline can be detected, typically about 2 to about 24 hours. Preferably, the reaction is performed under an inert atmosphere such as dry nitrogen.

Generally the reactants are combined stoichiometrically. When an amine base is used for those compounds where a salt (typically the HCI salt) of an amine 4 or 5 is used, it is preferable to use excess gamine base, generally an extra equivalent of amine base. (Alternatively, if an amine base is not used an excess of the amine 4 or 5 may be used).

For those compounds where a sterically hindered amine 4 (such as a 2-alkyl-3-ethynylaniline) or very reactive 4-haloquinazoline is used it is preferable to use t-butyl alcohol or a polar aprotic solvent such as DMF or N-methylpyrrolidin-2-one as the solvent.

Alternatively, a 4-substituted quinazoline 2 wherein X is hydroxyl or oxy (and the 2- nitrogen is hydrogenated) is reacted with carbon tetrachloride and an optionally substituted triarylphosphine which is optionally supported on an inert polymer (e.g. triphenylphosphine, polymer supported. Aldrich Cat. No. 36,645-5, which is a 2% divinylbenzene cross-linked polystyrene containing 3 mmol phosphorous per gram resin) in a solvent such as carbon tetrachloride, chloroform, dichloroethane, tetrahydrofuran, acetonitrile or other aprotic solvent or mixtures thereof. The reaction mixture is maintained at a temperature from about ambient to reflux, preferably from about 35°C to reflux, for 2 to 24 hours. This mixture is reacted with the appropriate amine or amine hydrochloride 4 or 5 either directly or after removal of solvent, for example by vacuum evaporation, and addition of a suitable alternative solvent such as a (C₁-C₆) alcohol, DMF, N-methylpyrrolidin-2-one, pyridine or 1-4 dioxane. Then, the reaction mixture is maintained at a temperature from about ambient to the reflux temperature of the solvent preferably from about 35°C to about reflux, until substantially complete formation of product is acheived, typically from about 2 to about 24 hours. Preferably the reaction is performed under an inert atmosphere such as dry nitrogen.

When compound 4, wherein Y is Br, I, or trifluoromethanesulfonyloxy, is used as starting material in the reaction with quinazoline 2, a compound of formula 3 is formed wherein R¹, R², R³, and Y are as described above. Compound 3 is converted to compounds of formula 1 wherein R⁴ is R¹¹ ethynyl, and R¹¹ is as defined above, by reaction with a suitable palladium reagent such as tetrakis(triphenylphosphine)palladium or bis(triphenylphosphine)palladium dichloride in the presence of a suitable Lewis acid such as cuprous chloride and a suitable alkyne such as trimethylsilylacetylene, propargyl alcohol or 3-(N,N-dimethylamino)-propyne in a solvent such as diethylamine or triethytamine. Compounds 3, wherein Y is NH₂, may be converted to compounds 1 wherein R⁴ is azide by treatment of compound 3 with a diazotizing agent, such as an acid and a nitrite (e.g., acetic acid and NaNO₂) followed by treatment of the resulting product with an azide, such as NaN₃.

For the production of those compounds of Formula I wherein an R¹ is an amino or hydroxyamino group the reduction of the corresponding Formula I compound wherein R¹ is nitro is employed.

The reduction may conveniently be carried out by any of the many procedures known for such transformations. The reduction may be carried out, for example, by hydrogenation of the nitro compound in a reaction-inert solvent in the presence of a suitable metal catalyst such as palladium, platinum or nickel. A further suitable reducing agent is, for example, an activated metal such as activated iron (produced by washing iron powder with a dilute solution of an acid such as hydrochloric acid). Thus, for example, the reduction may be carried out by heating a mixture of the nitro compound and the activated metal with concentrated hydrochloric acid in a solvent such as a mixture of water and an alcohol, for example, methanol or ethanol, to a temperature in the range, for example, 50 to 150°C, conveniently at or near 70°C. Another suitable class of reducing agents are the alkali metal dithionites, such as sodium dithionite, which may be used in (C₁-C₄)alkanoic acids, (C₁-C₆)alkanols, water or mixtures thereof.

For the production of those compounds of Formula I wherein R² or R³ incorporates a primary or secondary amino moiety (other than the amino group intended to react with the quinazoline), such free amino group is preferably protected prior to the above described reaction followed by deprotection, subsequent to the above described reaction with 4-(substituted)quinazoline 2.

Several well known nitrogen protecting groups can be used. Such groups include (C₁-C₆)alkoxycarbonyl, optionally substituted benzyloxycarbonyl, aryloxycarbonyl, trityl, vinyloxycarbonyl, O-nitrophenylsulionyl, diphenylphosphinyl, p-toluenesuffonyl, and benzyl. The addition of the nitrogen protecting group may be carried out in a chlorinated hydrocarbon solvent such as methylene chloride or 1,2-dichloroethane, or an ethereal solvent such as glyme, diglyme or THF, in the presence or absence of a tertiary amine base such as triethylamine, diisopropylethylamine or pyridine, preferably triethylamine, at a temperature from about 0°C to about 50°C, preferably about ambient temperature. Alternatively, the protecting groups are conveniently attached using Schotten-Baumann conditions.

Subsequent to the above described coupling reaction, of compounds 2 and 5, the protecting group may be removed by deprotecting methods known to those skilled in the art such as treatment with trifluoroacetic acid in methylene chloride for the tert-butoxycarbonyl protected products.

For a description of protecting groups and their use, see T.W. Greene and P.G.M. Wuts, "Protective Groups in Organic Synthesis" Second Ed., John Wiley & Sons, New York, 1991.

For the production of compounds of Formula I wherein R¹ or R² is hydroxy, cleavage of a Formula I compound wherein R¹ or R² is (C₁-C₄)alkoxy is preferred.

The cleavage reaction may conveniently be carried out by any of the many procedures known for such a transformation. Treatment of the protected formula I derivative with molten pyridine hydrochloride (20-30 eq.) at 150 to 175°C may be employed for O-dealkylations. Alternatively, the cleavage reaction may be carried out, for example, by treatment of the protected quinazoline derivative with an alkali meta! (C₁₋C₄)alkylsulphide, such as sodium ethanethiolate or by treatment with an alkali metal diarylphosphide such as lithium diphenylphosphide. The cleavage reaction may also, conveniently, be carried out by treatment of the protected quinazoline derivative with a boron or aluminum trihalide such as boron tribromide. Such reactions are preferably carried out in the presence of a reaction-inert solvent at a suitable temperature.

Compounds of formula I, wherein R¹ or R² is a (C₁-C₄)alkylsulphinyl or (C₁-C₄)alkylsulphonyl group are preferably prepared by oxidation of a formula I compound wherein R¹ or R² is a (C₁-C₄)alkylsulfanyl group. Suitable oxidizing agents are known in the art for the oxidation of sulfanyl to sulphinyl and/or sulphonyl, e. g., hydrogen peroxide, a peracid (such as 3-chloroperoxybenzoic or peroxyacetic acid), an alkali metal peroxysulphate (such as potassium peroxymonosulphate), chromium trioxide or gaseous oxygen in the presence of platinum. The oxidation is generally carried out under as mild conditions as possible using the stoichiometric amount of oxidizing agent in order to reduce the risk of over oxidation and damage to other functional groups. In general, the reaction is carried out in a suitable solvent such as methylene chloride, chloroform, acetone, tetrahydrofuran or tert-butyl methyl ether and at a temperature from about -25 to 50°C, preferably at or near ambient temperature, i. e., in the range of 15 to 35°C. When a compound carrying a sulphinyl group is desired a milder oxidizing agents should be used such as sodium or potassium metaperiodate, conveniently in a polar solvent such as acetic acid or ethanol. The compounds of formula I containing a (C₁-C₄)alkylsulphonyl group may be obtained by oxidation of the corresponding (C₁-C₄)alkylsulphinyl compound as well as of the corresponding (C,-C₄)alkylsulfanyl compound.

Compounds of formula I wherein R¹ is optionally substituted (C₂-C₄)alkanoylamino, ureido, 3-phenylureido, benzamido or sulfonamide can be prepared by acylation or sulfonylation of a corresponding compound wherein R¹ is amino. Suitable acylating agents are any agents known in the art for the acylation of amino to acylamino, for example, acyl halides, e.g., a (C₂-C₄)alkanoyl chloride or bromide or a benzoyl chloride or bromide, alkanoic acid anhydrides or mixed anhydrides (e.g., acetic anhydride or the mixed anhydrides formed by the reaction of an alkanoic acid and a (C,-C₄)alkoxycarbonyl halide, for example (C₁-C₄)alkoxycarbonyl chloride, in the presence of a suitable base. For the production of those compounds of Formula I wherein R¹ is ureido or 3-phenylureido, a suitable acylating agent is, for example, a cyanate, e.g., an alkali metal cyanate such as sodium cyanate, or an isocyanate such as phenyl isocyanate. N-sulfonylations may be carried out with suitable sulfonyl halides or sulfonylanhydrides in the presence of a tertiary amine base. In general the acylation or sulfonylation is carried out in a reaction-inert solvent and at a temperature in the range of about -30 to 120°C, conveniently at or near ambient temperature.

Compounds of Formula I wherein R¹ is (C₁-C₄)alkoxy or substituted (C,-C₄)alkoxy or R¹ is (C₁-C₄)alkylamino or substituted mono-N- or di-N,N-(C,-C₄)alkylamino, are prepared by the alkylation, preferably in the presence of a suitable base, of a corresponding compound wherein R¹ is hydroxy or amino, respectively. Suitable alkylating agents include alkyl or substituted alkyl halides, for example, an optionally substituted (C₁-C₄)alkyl chloride, bromide or iodide, in the presence of a suitable base in a reaction-inert solvent and at a temperature in the range of about 10 to 140°C, conveniently at or near ambient temperature.

For the production of those compounds of Formula I wherein R¹ is an amino-, oxy- or cyano-substituted (C₁-C₄)alkyl substituent, a corresponding compound wherein R¹ is a (C₁-C₄)alkyl substituent bearing a group which is displacable by an amino-, alkoxy-, or cyano group is reacted with an appropriate amine, alcohol or cyanide, preferably in the presence of a suitable base. The reaction is preferably carried out in a reaction-inert solvent or diluent and at a temperature in the range of about 10 to 100°C, preferably at or near ambient temperature.

Compounds of Formula I, wherein R¹ is a carboxy substituent or a substituent which includes a carboxy group are prepared by hydrolysis of a corresponding compound wherein R¹ is a (C₁-C₄)alkoxycarbonyl substituent or a substituent which includes a (C₁-C₄)alkoxycarbonyl group. The hydrolysis may conveniently be performed, for example, under basic conditions, e.g., in the presence of alkali metal hydroxide as illustrated in the accompanying Examples.

Compounds of Formula I wherein R¹ is amino, (C₁-C₄)alkylamino, di-[(C₁-C₄)alkyl]amino, pyrrolidin-1-yl, piperidino, morpholino, piperazin-1-yl, 4-(C₁-C₄)alkylpiperazin-1-yl or (C₁-C₄)alkysulfanyl, may be prepared by the reaction, in the presence of a suitable base, of a corresponding compound wherein R¹ is an amine or thiol displaceable group with an appropriate amine or thiol. The reaction is preferably carried out in a reaction-inert solvent or diluent and at a temperature in the range of about 10 to 180°C, conveniently in the range 100 to 150°C.

Compounds of Formula I wherein R¹ is 2-oxopyrrolidin-1-yl or 2-oxopiperidin-1-yl are prepared by the cyclisation, in the presence of a suitable base, of a corresponding compound wherein R¹ is a halo-(C₂-C₄)alkanoylamino group. The reaction is preferably carried out in a reaction-inert solvent or diluent and at a temperature in the range of about 10 to 100°C, conveniently at or near ambient temperature.

For the production of compounds of Formula I in which R¹ is carbamoyl, substituted carbamoyl, alkanoyloxy or substituted alkanoyloxy, the carbamoylation or acylation of a corresponding compound wherein R¹ is hydroxy is convenient.

Suitable acylating agents known in the art for acylation of hydroxyaryl moieties to alkanoyloxyaryl groups include, for example, (C₂-C₄)alkanoyl halides, (C₂-C₄)alkanoyl anhydrides and mixed anhydrides as described above, and suitable substituted derivatives thereof may be employed, typically in the presence of a suitable base. Alternatively, (C²-C₄)alkanoic acids or suitably substituted derivatives thereof may be coupled with a Formula I compound wherein R¹ is hydroxy with the aid of a condensing agent such as a carbodiimide. For the production of those compounds of Formula I in which R¹ is carbamoyl or substituted carbamoyl, suitable carbamoylating agents are, for example, cyanates or alkyl or arylisocyanates, typically in the presence of a suitable base. Alternatively, suitable intermediate such as the chloroformate or carbonylimidazolyl derivative of a compound of Formula I in which R¹ is hydroxy may be generated, for example, by treatment of said derivative with phosgene (or a phosgene equivalent) or carbonyldiimidazole. The resulting intermediate may then be reacted with an appropriate amine or substituted amine to produce the desired carbamoyl derivatives.

Compounds of formula I wherein R¹ is aminocarbonyl or a substituted aminocarbonyl can be prepared by the aminolysis of a suitable intermediate in which R¹ is carboxy.

The activation and coupling of formula I compounds wherein R¹ is carboxy may be performed by a variety of methods known to those skilled in the art. Suitable methods Include activation of the carboxyl as an acid halide, azide, symmetric or mixed anhydride, or active ester of appropriate reactivity for coupling with the desired amine. Examples of such types of intermediates and their production and use in couplings with amines may be found extensively In the literature; for example M. Bodansky and A. Bodansky, "The Practice of Peptide Synthesis", Springer,-Verlag, New York, 1984. The resulting formula I compounds may be isolated and purified by standard methods, such as solvent removal and recrystallization or chromatography.

The starting materials for the above described reaction schemes (e.g., amines, quinazolines and amine protecting groups) are readily available or can be easily synthesized by those skilled In the art using conventional methods of organic synthesis. For example, the preparation of 2,3-dihydro-1,4-benzoxazine derivatives are described in R. C. Elderfield, W.H. Todd, S. Gerber, Ch. 12 in "Heterocyclic Compounds", Vol. 6, R. C. Elderfield ed., John Wiley and Sons, Inc., N.Y., 1957. Substituted 2,3-dihydrobenzothiazinyl compounds are described by R.C. Elderfield and E.E. Harris in Ch. 13 of Volume 6 of the Elderfield "Heterocyclic Compounds" book.

Certain Formula I quinazolines can exist in solvated, as well as unsolvated forms, such as the hydrated forms.

A suitable pharmaceutically-acceptable salt of a compound of formula I is, for example, an acid-addition salt of a corresponding compound which is sufficiently basic, e.g., an acid-addition salt with, for example, an inorganic or organic acid such as hydrochloric, hydrobromic, sulphuric, phosphoric, methanesulfonic, benzenesulfonic, trifluoroacetic, citric, lactic or maleic acid. A suitable pharmaceutically-acceptable base-addition salt of a compound of formula I which is acidic is an alkali metal salt, for example, a lithium, sodium or potassium salt; an a!kaline earth metal salt, for example, a calcium or magnesium salt; an ammonium salt; or a salt with an organic base which affords a physiologically-acceptable cation for example a salt with methylamine, dimethylamine, trimethylamine, piperidine, morpholine or tris-(2-hydroxyethyl)amine. All such salts can be prepared by conventional methods. For example, they can be prepared simply by contacting the acidic and basic entities, usually in a stoichiometric ratio, in either an aqueous, nonaqueous or partially aqueous mediuni, as appropriate. The salts are recovered by filtration: by precipitation with a non-solvent, preferably an etheral or hydrocarbon solvent, followed by filtration and by evaporation of a solvent, or, in the case of aqueous solutions, by lyophilization.

Some of the compounds of Formula I have asymmetric carbon atoms. Such diasteromeric mixtures can be separated into their individual diastereomers on the basis of their physical chemical difference by methods known per se., for example, by chromatography and/or fractional crystallization. Enantiomers can be separated by converting the enantiomeric mixtures into a diastereomric mixture by reaction with an appropriate optically active compound (e.g., alcohol), separating the diastereomers and converting (e.g., hydrolyzing) the Individual diastereomers to the corresponding pure enantiomers.

The active compounds are potent inhibitors of the erbB family of oncogenic and protooncogenic protein tyrosine kinases such as epidermal growth factor receptor (EGFR), erbB2, HER3, or HER4 and thus, are all adapted to therapeutic use as antiproliferative agents (e.g., anticancer) in mammals, particularly humans. In particular, the compounds are therapeutants or prophylactics for the treatment of a variety of human tumors (renal, liver, kidney, bladder, breast, gastric, ovarian, colorectal, prostate, pancreatic, lung, vulval, thyroid, hepatic carcinomas, sarcomas, glioblastomas, various head and neck tumors), and other hyperplastic conditions such as benign hyperplasia of the skin (e.g., psoriasis) or prostate (e.g., BPH). It is, in addition, expected that a quinazoline as described above may possess activity against a range of leukemias and lymphoid malignancies.

The active compounds may also be expected to be useful in the treatment of additional disorders in which aberrant expression ligand/receptor interactions, activation or signalling events related to various protein tyrosine kinases, whose activity is inhibited by the agents of Formula I, are involved.

Such disorders may include those of neuronal, glial, astrocytal, hypothalamic, and other glandular, macrophagal, epithelial, stromal, and blastocoelic nature in which aberrant function, expression, activation or signalling of the erbB tyrosine kinases may be involved. In addition, compounds of Formula I may have therapeutic utility in inflammatory, angiogenic and immunologic disorders involving both identified and as yet unidentified tyrosine kinases which are inhibited by compounds of Formula I.

The in vitro activity of the active compounds in inhibiting the receptor tyrosine kinase (and thus subsequent proliferative response, e.g., cancer) may be determined by the procedure detailed below.

Activity of the active compunds, in vitro, can be determined by the amount of inhibition of the phosphorylation of an exogenous substrate (e.g., Lys₃ - Gastrin or polyGluTyr (4:1) random copolymer (I. Posner et. al., J. Biol. Chem. 267 (29). 20638-47 (1992)) on tyrosine by epidermal growth factor receptor kinase by a test compound relative to a control. Affinity purified, soluble human EGF receptor (96 ng) is obtained according to the procedure in G. N. Gill, W. Weber, Methods in Enzymology 146, 82-88 (1987) from A431 cells (American Type Culture Collection, Rockville, MD) and preincubated in a microfuge tube with EGF (2µg/ml) in phosphorylation buffer + vanadate (PBV: 50 mM HEPES, pH 7.4; 125 mM NaCI; 24 mM MgCl₂; 100µM sodium orthovanadate), in a total volume of 10 µl, for 20-30 minutes at room temperature. The test compound, dissolved in dimethylsulfoxide (DMSO), is diluted in PBV, and 10 µl is mixed with the EGF receptor /EGF mix, and incubated for 10-30 minutes at 30°C. The phosphorylation reaction is initiated by addition of 20 µl ³³P-ATP/ substrate mix (120 µM Lys₃-Gastrin (sequence in single letter code for amino acids, KKKGPWLEEEEEAYGWLDF), 50 mM Hepes pH 7.4, 40 µM ATP, 2 µCi γ-[³³P]-ATP) to the EGFr/EGF mix and incubated for 20 minutes at room temperature. The reaction is stopped by addition of 10 µl stop solution (0.5 M EDTA, pH 8; 2mM ATP) and 6 Il 2N HCI. The tubes are centrifuged at 14,000 RPM, 4°C, for 10 minutes. 35 µl of supernatant from each tube is pipetted onto a 2.5 cm circle of Whatman P81 paper, bulk washed four times in 5% acetic acid, 1 liter per wash, and then air dried. This results in the binding of substrate to the paper with loss of free ATP on washing. The [³³P] incorporated is measured by liquid scintillation counting. Incorporation in the absence of substrate (e.g., lys₃-gastrin) is subtracted from all values as a background and percent inhibition is calculated relative to controls without test compound present.

Such assays, carried out with a range of doses of test compounds, allow the determination of an approximate IC₅₀ value for the in vitro inhibition of EGFR kinase activity. Although the inhibitory properties of the compounds of Formula I vary with structural change as expected, the activity generally exhibited by these agents, determined in the manner described above, is in the range of IC₅₀=0.0001-30 µM.

Activity of the active compounds, in vivo, can be determined by the amount of inhibition of tumor growth by a test compound relative to a control. The tumor growth inhibitory effects of various compounds are measured according to the methods of Corbett T. H., et al. "Tumor Induction Relationships in Development of Transplantable Cancers of the Colon in Mice for Chemotherapy Assays, with a Note on Carcinogen Structure", Cancer Res., 35, 2434-2439 (1975) and Corbett, T. H., et al., "A Mouse Colon-tumor Model for Experimental Therapy", Cancer Chemother. Rep. (Part 2)", 5, 169-186 (1975), with slight modifications. Tumors are induced in the left flank by s.c. injection of 1 X 10⁸ log phase cultured tumor cells (human MDA-MB-468 breast or human HN5 head and neck carcinoma cells) suspended in 0.10 ml RPMI 1640. After sufficient time has elapsed for the tumors to become palpable (2-3 mm in diameter) the test animals (athymic mice) are treated with active compound (formulated by dissolution in DMSO typically at a concentration of 50 to 100 mg/mL followed by 1:9 dilution into saline or, alternatively, 1:9 dilution into 0.1% Pluronic^{®} P105 in 0.9% saline) by the intraperitoneal (ip) or oral (po) routes of administration twice daily (i.e., every 12 hours) for 5 consecutive days. In order to determine an anti-tumor effect, the tumor is measured in millimeters with Vernier calipers across two diameters and the tumor size (mg) is calculated using the formula: Tumor weight = (length x [width]²)/2, according to the methods of Geran, R.I., et al. "Protocols for Screening Chemical Agents and Natural Products Against Animal Tumors and Other Biological Systems. Third Edition, Cancer Chemother. Rep., 3, 1-104 (1972). Results are expressed as percent inhibition, according to the formula: Inhibition (%) = (TuW_{control} - TuWₜₑₛₜ)/TuW_{control} x 100%. The flank site of tumor implantation provides reproducible dose/response effects for a variety of chemotherapeutic agents, and the method of measurement (tumor diameter) is a reliable method for assessing tumor growth rates.

Administration of the active compounds can be effected by any method which enables delivery of the compounds to the site of action (e.g., cancer cells). These methods include oral routes, intraduodenal rou'...s, parenteral injection (including intravenous, subcutaneous, intramuscular, intravascular or infusion), topical administration, etc.

The amount of active compound administered will, of course, be dependent on the subject being treated, on the severity of the affliction, on the manner of administration and on the judgement of the prescribing physician. However an effective dosage is in the range of approximately 0.001-100 mg/kg, preferably 1 to 35 mg/kg in single or divided doses. For an average 70kg human, this would amount to 0.05 to 7 g/day, preferably 0.2 to 2.6 g/day.

The composition may, for example, be in a form suitable for oral administration as a tablet, capsule, pill, powder, sustained release formulations, solution, suspension, for parenteral injection as a sterile solution, suspension or emulsion, for topical administration as an ointment or cream or for rectal administration as a suppository. The pharmaceutical composition may be in unit dosage forms suitable for single administration of precise dosages. The pharmaceutical composition will include a conventional pharmaceutical carrier or excipient and a compound according to the invention as an active Ingredient. In addition, it may include other medicinal or pharmaceutical agents, carriers, adjuvants, etc.

Pharmaceutical compositions may contain 0.1%-95% of the compound, preferably 1%-70%. In any event, the composition or formulation to be administered will contain a quantity of active compound in an amount effective to alleviate or reduce the signs in the subject being treated, i.e., hyperproliferative diseases, over the course of the treatment.

Exemplary parenteral administration forms include solutions or suspensions of active compounds In sterile aqueous solutions, for example aqueous propylene glycol or dextrose solutions. Such dosage forms can be suitably buffered, if desired.

Suitable pharmaceutical carriers include inert diluents or fillers, water and various organic solvents. The pharmaceutical compositions may, if desired, contain additional ingredients such as flavorings, binders, excipients and the like. Thus, for oral administration, tablets containing various excipients, such as citric acid may be employed together with various disintegrants such as starch, alginic acid and certain complex silicates and with binding agents such as sucrose, gelatin and acacia. Additionally, lubricating agents such as magnesium stearate, sodium lauryl sulfate and talc are often useful for tableting purposes. Solid compositions of a similar type may also be employed in soft and hard filled gelatin capsules. Preferred materials, therefor, include lactose or milk sugar and high molecular weight polyethylene glycols. When aqueous suspensions or elixirs are desired for oral administration the active compound therein may be combined with various sweetening or flavoring agents, coloring matters or dyes and, if desired, emulsifying agents or suspending agents, together with diluents such as water, ethanol, propylene glycol, glycerin, or combinations thereof.

Methods of preparing various pharmaceutical compositions with a specific amount of active compound are known, or will be apparent, to those skilled in this art. For examples, see Remington's Pharmaceutical Sciences., Mack Publishing Company, Easter, Pa., 15th Edition (1975).

The hyperproliferative disease treatment described above may be applied as a sole therapy or may involve, in addition to the active compound, one or more other antitumor substances. Such conjoint treatment may be achieved by way of the simultaneous, sequential, cyclic or separate dosing of the individual components of the treatment.

High pressure liquid chromatography (HPLC) used in the following examples and preparations was effected according to the following method unless modified in specific examples. Perkin-Elmer Pecosphere" 3X3C cartridge column (3mm X 3cm, C18; available from Perkin Elmer Corp., Norwalk, CT 06859) with a Brownlee (trademark) RP-8 Newguard precolumn (7 micron, 3.2mm X 15mm, available from Applied Biosystems Inc. San Jose, CA 95134) which was previously equilibrated in pH 4.50, 200 mM ammonium acetate buffer. Samples were eluted using a linear gradient of 0-100% acetonitrile/pH4.50, 200 mM NH₄ acetate over 10 minutes with a flow rate of 3.0 mL/min. Chromatograms were generated over the range 240-400nm using a diode array detector.

It should be understood that the scope of the invention is as defined by the claims.

### PREPARATION 1

### 6,7-Bis(2-methoxy-ethoxy)-quinazolone

To ethyl 3,4-dihydroxybenzoate (36.4 g. 0.200 mol), K₂CO₃ (60.8 g. 0.44 mol) and tetrabutylammonium iodide (750 mg) in degassed acetone (400 mL) was added 2-bromoethyl methyl ether (69.5 g, 47 mL). The mixture was stirred under N₂ at reflux for 64 hours. Ether (600 mL) was added to the mixture and after stirring 30 minutes at 20°C the precipitated salts were removed by filtration. The filtrate was concentrated in vacuo and the residue was triturated with hexane (500 mL) for 30 minutes and the white solid ethyl 3,4-bis(2-methoxy-ethoxy)benzoate was filtered and dried in vacuo (55.5 g; 93%; M.P. 50-51 °C). A portion of this product (45.7 g, 0.158 mol) in acetic acid (150 mL) was treated dropwise with cone. HNO₃ (40 mL) at 5°C and the solution stirred 24 hours before pouring into cold H₂O (1.6 L). The mixture was extracted with ethyl acetate (1.1 L), and the organic phase was washed three times with 200 mL H₂O, and brine, dried over Na₂SO₄, filtered and concentrated in vacuo to afford ethyl 4,5-bis-(2-methoxy-ethoxy)-2-nitro-benzoate (54.3 g) as a brown oil. This nitro product (52.0 g. 0.15 mol) was dissolved in ethanol (1000 mL) containing 1 equivalent of HCl (generated In the ethanol by prior addition of 11 mL acetyl chloride), PtO₂•H₂O (1.0 g) was added, and the mixture was hydrogenated under 45 psi H₂ for 6 hours. The catalyst was removed by filtration through Celile, and the filtrate was concentrated in vacuo to a thick slurry which was diluted with ether (400 mL). The solid white hydrochloride salt of ethyl 2-amino-4,5-bis-(2-methoxy-ethoxy)benzoate was filtered and dried in vacuo (44.7 g; 88%). A portion of this material (42 g, 0.12 mol) and ammonium formate (7.6 g. 0.12 mol) were disssolved in formamide (63 mL) and the stirred mixture was heated to 160-165 °C under an atmosphere of N₂ for 3 hours. H₂O (200 mL) was added and after cooling the precipitated crude title product was recovered by filtration, washed with cold H₂O, and dried in vacuo. The filtrate was extracted five times with CHCl_{3,} and the pooled organic extracts were washed with brine, dried over Na₂SO₄, and concentrated in vacuo. The residue and crude quinazolone precipitate were combined, triturated in hot acetonitrile (250 mL) for 30 minutes, cooled to 20°C and treated with ether (250 mL). After cooling to 4 °C the white solid was filtered and dried in vacuo (30.4 g, 86%; GC-MS m/z 294 (M⁺)).

### PREPARATION 2

### 4-Chloro-6,7-bis-(2-methoxy-ethoxy)-quinazoline

To 6,7-bis(2-methoxy-ethoxy)-quinazolone (500 mg,1.7 mmol), from Preparation 1, in CHCl₃ (10 mL) containing one drop of DMF was added oxalylchloride (490µL, 5.6 mmol) in several portions over 5 minutes. Once foaming ceased the solution was refluxed 1.5 hours. The solvent was removed in vacuo and the residue was dissolved In 1,2-dichloroethane (20 mL) and washed two times with 80 mL saturated aqueous Na₂CO₃. The organic phase was dried over Na₂SO₄, and concentrated in vacuo to afford solid title product (520 mg, 92%; M.P. 108-109 °C).

### PREPARATION 3

### 4-Chloro-6,7-bis-(2-chloro-ethoxy)-quinazoline*, 4-chloro-6-(2-chloro-ethoxy)-7-(2-methoxy-ethoxy)-quinazoline* and 4-chloro-6,7-bis-(2-methoxy-ethoxy)-quinazoline and 4-chloro-7-(2-chloro-ethoxy)-6-(2-methoxy-ethoxy)-quinazoline*

6,7-Bis(2-methoxy-ethoxy)-quinazolone (5.4 g, 18.3 mmol), from Preparation 1, and pyridine (3.0 mL, 37 mmol) were heated in refluxing POCl₃ (22 mL) under an atmosphere of dry nitrogen for 2.5 hours. Following concentration of the mixture in vacuo at 60°C the residue was dissolved in CHCl₃ (150 mL) and carefully added in portions with stirring to cold saturated aqueous NaHCO₃ (100 mL). The mixture was stirred 10 min. after the addition was complete and the organic phase was separated, washed with brine, dried over Na₂SO₄, and concentrated in vacuo. The residue was flash chromatographed on silica using a gradient of 20% to 60% ethyl acetate/hexanes to afford 3.41 g of 4-chloro-6.7-bis-(2-methoxy-ethoxy)-quinazoline, 234 mg of 4-chloro-6-(2-chloro-ethoxy)-7-(2-methoxy-ethoxy)-quinazoline, 532 mg of 4-chloro-7-(2-chloro-ethoxy)-6-(2-methoxy-ethoxy)-quinazoline, and 330 mg of 4-chloro-6,7-bis-(2-chloro-ethoxy)-quinazoline.
* Not within scope of appended claims

## Claims

1. A compound selected from the compounds of the formula: 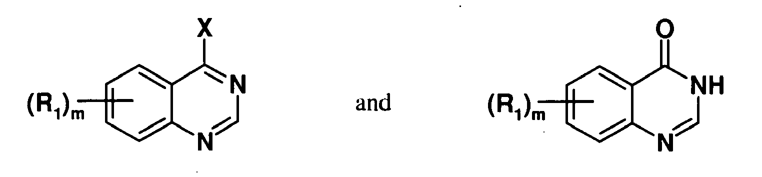 wherein
X is OH;
m is 2 or 3;
each R₁ is R₅O, where R₅ is (C₁-C₄) alkyl; wherein the alkyl moiety in R₅O is substituted with R₆O, where R₆ is hydrogen or R₅ and the compound 4-chloro-6,7-bis-(2-methoxy-ethoxy)-quinazoline.

2. The compound of claim 1, having the formula 4-chloro-6,7-bis-(2-methoxy-ethoxy)-quinazoline.

3. The compound of claim 1, having the structure: 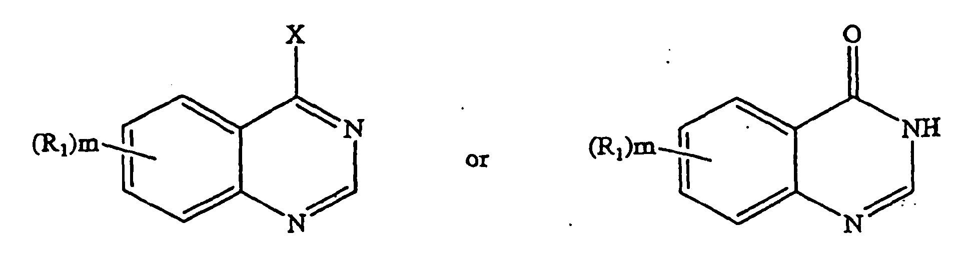 wherein X is OH, and R₁ and m are as defined above.

4. The compound of claim 3, wherein m is 2.

5. The compound of claim 4, wherein each R₁ is 2-methoxy-ethoxy.

6. A process for preparing the compound 4-Chloro-6,7-bis-(2-methoxy-ethoxy)-quinazoline, which comprises treating the compound having the structure: 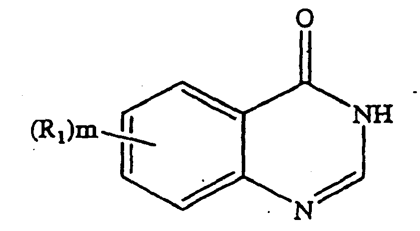 wherein m is 2 and each R₁ is 2-methoxy-ethoxy, with oxalylchloride in the presence of CHCl₃ and DMF.

## Patentansprüche

1. Verbindung ausgewählt aus den Verbindungen der Formel: 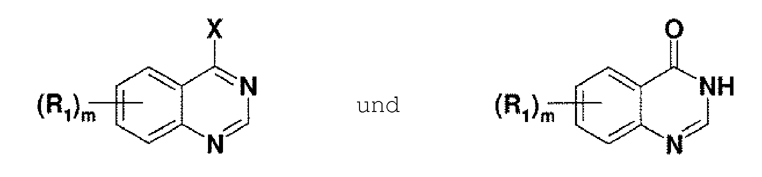 wobei
X für OH steht;
m für 2 oder 3 steht;
R₁ jeweils für R₅O steht, wobei R₅ für (C₁-C₄) -Alkyl steht; wobei die Alkylgruppe in R₅O durch R₆O substituiert ist, wobei R₆ für Wasserstoff oder R₅ steht, und der Verbindung 4-Chlor-6,7-bis-(2-methoxy-ethoxy)-chinazolin.

2. Verbindung nach Anspruch 1 mit der Formel 4-Chlor-6,7-bis-(2-methoxy-ethoxy)-chinazolin.

3. Verbindung nach Anspruch 1 mit der Struktur: 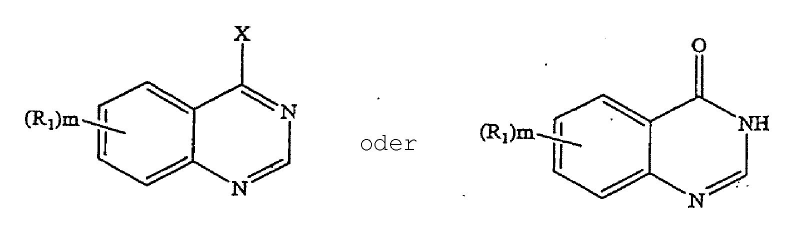 wobei X für OH steht und R₁ und m wie oben definiert sind.

4. Verbindungen nach Anspruch 3, wobei m für 2 steht.

5. Verbindungen nach Anspruch 4, wobei R₁ jeweils für 2-Methoxy-ethoxy steht.

6. Verfahren zur Herstellung der Verbindung 4-Chlor-6,7-bis-(2-methoxy-ethoxy)-chinazolin, bei dem man die Verbindung der Struktur 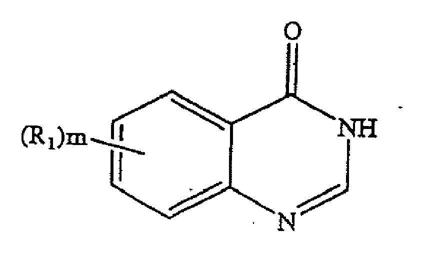 in welcher m für 2 steht und R₁ jeweils für 2-Methoxy-ethoxy steht, in Gegenwart von CHCl₃ und DMF mit Oxalsäurechlorid behandelt.

## Revendications

1. Composé choisi parmi les composés de formule : 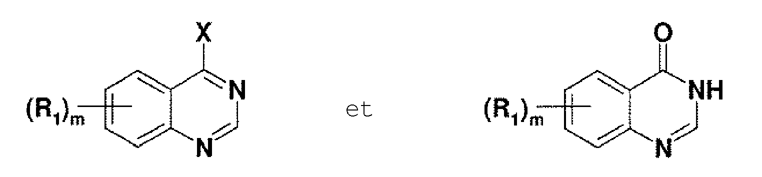 dans lesquelles
X est OH ;
m est 2 ou 3 ;
chaque R₁ est R₅O, où R₅ est alkyle en (C₁-C₄) ; le motif alkyle dans R₅O étant substitué par R₆O, où R₆ est hydrogène ou R₅ et le composé 4-chloro-6, 7-bis-(2-méthoxy-éthoxy)-quinazoline.

2. Composé de la revendication 1, ayant la formule 4-chloro-6,7-bis-(2-méthoxy-éthoxy)-quinazoline.

3. Composé de la revendication 1, ayant la structure : 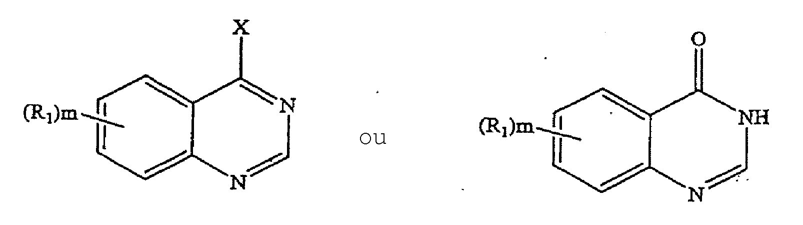 dans lesquelles X est OH, et R₁ et m sont tels que définis ci-dessus.

4. Composé de la revendication 3, **caractérisé en ce que** m est 2.

5. Composé de la revendication 4, **caractérisé en ce que** chaque R₁ est 2-méthoxy-éthoxy.

6. Procédé de préparation du composé 4-chloro-6, 7-bis-(2-méthoxy-éthoxy)-quinazoline, **caractérisé en ce qu'**il comprend le traitement du composé ayant la structure : 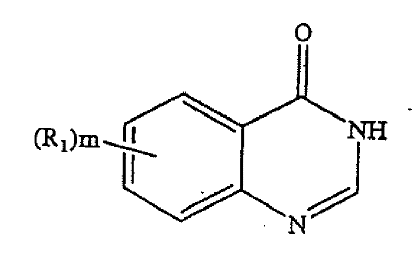 dans laquelle m est 2 et chaque R₁ est 2-méthoxy-éthoxy, avec du chlorure d'oxalyle en présence de CHCl₃ et de DMF.
